(19) Europäisches Patentamt
European Patent Office
Office européen des brevets

(11) **EP 1 916 913 B1**

## (12) EUROPÄISCHE PATENTSCHRIFT

(45) Veröffentlichungstag und Bekanntmachung des Hinweises auf die Patenterteilung:
**10.11.2010 Patentblatt 2010/45**

(21) Anmeldenummer: 06778335.7

(22) Anmeldetag: **23.08.2006**

(51) Int Cl.:
*A23L 1/305* (2006.01)      *A61P 3/10* (2006.01)

(86) Internationale Anmeldenummer:
**PCT/EP2006/065611**

(87) Internationale Veröffentlichungsnummer:
**WO 2007/023172 (01.03.2007 Gazette 2007/09)**

(54) **PROTEINZUSAMMENSETZUNG ZUR BEHANDLUNG EINES PHYSIOLOGISCH BEDINGTEN, KLINISCH UNAUFFÄLLIGEN PROTEINMEHRBEDARFS**

PROTEIN COMPOSITION FOR TREATING A PHYSIOLOGICALLY CAUSED, CLINICALLY NORMAL INCREASED NEED FOR PROTEIN

COMPOSITION A BASE DE PROTEINES POUR TRAITER DES BESOINS SUPPLEMENTAIRES EN PROTEINES, INHERENTS A LA PHYSIOLOGIE ET NON APPARENTS SUR LE PLAN CLINIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **23.08.2005 EP 05018276**

(43) Veröffentlichungstag der Anmeldung:
**07.05.2008 Patentblatt 2008/19**

(73) Patentinhaber: **Herberger, Dr., Armand 67435 Neustadt an der Weinstrasse (DE)**

(72) Erfinder: **Herberger, Dr., Armand 67435 Neustadt an der Weinstrasse (DE)**

(74) Vertreter: **Betten & Resch Patentanwälte Theatinerstrasse 8 80333 München (DE)**

(56) Entgegenhaltungen:
**EP-A- 0 482 715      EP-A- 1 479 300
WO-A-02/087562      WO-A-2004/049830
WO-A-2004/058243      US-A- 5 242 697
US-A1- 2004 063 608**

EP 1 916 913 B1

**Beschreibung**

Die Erfindung betrifft eine diätetische Zusammensetzung aufder Basis von Proteinen für die physiologische Behandlung von Patienten, Insbesondere betrifft die Erfindung eine diätetische Zusammensetzung zur Behandlung von physiologischen Proteinmangetzuständen umfassend eine Proteinzusammensetzung, enthaltend Proteine und bis zu 6 Gew.-% der kristallinen verzweigtkettigen Aminosäuren Isoleucin, Leucin und Valin, wobei der Gesamtgehalt der in der Proteinzusammensetzung enthaltenen verzweigtkettigen Aminosäuren Isoleucin, Leucin und Valin mindestens 27,5 Gew.-% bis maximal 40 Gew.-% beträgt, und wobei das Gewichtsverhältnis der verzweigtkettigen Aminosäuren Isoleucin: Leucin 1:1,4 bis 1:2,5 und das Gewichtsverhältnis der verzweigtkettigen Aminosäuren Isoleucin:Valin 1:1 bis 1:2 beträgt.

Erst in den letzten Jahren wird die Roile der Proteine bei der Ernährung von Patienten intensiver untersucht, um diese besser bei ihrer Ernährung zu unterstützen Dies gilt insbesondere für Patienten, die an Sarkopenie (Proteinmangelerscheinungen infolge Unterernährung), erhöhtem Gewicht info-lge Obesität, gestörtem Insulinstoffwechsel oder gestörten Leberproteinstoffwechsel leiden.

Sämtlichen Patienten wird dabei eine verbesserte Diät unter Gabe von mehr Protein und weniger Kohlenhydraten gegeben Durch die Verwendung von Protein in Diäten soll bei übergewichtigen Pattenten einerseits der Gewichtsverlust steigen und andererseits das Korperfett abnehmen, ohne dass es zu einem vermehrten Abbau der Muskelmasse kommt.

Das letztgenannte Problem tritt ublicherweise bei Patienten auf, die ihr Gewicht durch spezielle Diäten vermindern wollen, die einen hohen Anteil an Kohlenhydraten haben Hier kommt es zu einem Anstieg der Bluttriglyceride und gleichzeitig des Insulingehalts im Vergleich zu Diäten, die über einen hohen Proteingehalt verfugen

Infolgedessen müssen Diaten sorgfältig zwischen den Proteinen einerseits und den anderen Energietragern, namlich Fen und Kohlenhydraten andererseits eingestellt werden.

Neben Kohlehydraten und Fetten stellen aus $\alpha$-Aminosäuren aufgebaute Peptide und Proteine die dritte große Gruppe an Nahrungs- und Reservestoffen dar. Diese sind natürlich vorkommende Copolymere, die sich durch Kondensation von in der Regel 20 verschiedenen $\alpha$-Aminosäuren als Monomeren zusammensetzen. Peptide und Proteine unterscheiden sich in ihrer Größe. Ab ca. 100 Aminosäureresten spricht man meist von Proteinen, kleinere Moleküle werden als Peptide bezeichnet.

Die 20 verschiedenen $\alpha$-Aminosäuren, die in Proteinen vorkommen, werden als proteineigene Aminosäuren bezeichnet. Im Folgenden werden unter Aminosäuren immer proteineigene $\alpha$-Aminosäuren verstanden.

Pflanzen synthetisieren alle Aminosäuren aus einfachen Vorstufen, Menschen und Tiere dagegen können nur die nicht essentiellen Aminosäuren Alanin (Ala), Aspatat (Asp); Asparagin (Asn), Glutamat (Glu), Glutamin (Gln), Glycin (Gly), Omithin (Orn), Prolin (Pro) und Serin (Ser) selbst synthetisieren. Die essentiellen Aminosäuren Isoleucin, (Ile), Leucin (Leu), Lysin (Lys) Methionin (Met), Phenylalanin (Phe), Threonin (Thr), Tryptophan (Trp) und Valin (Val) müssen über die Nahrung zugeführt werden. Als semiessentiell gelten Arginin (Arg) und Histidin (His), die nur in Wachstumsphasen und bei Mangelerscheinungen von Außen zugeführt werden müssen. Eine Sonderstellung nehmen Cystein (Cys) und Tyrosin (Tyr) ein, die aus den essentiellen Aminosäuren Met und Phe synthetisiert werden können.

Die wichtigste Funktion der Aminosäuren ist die als Bausteine für die Biosynthese der Proteine. Fehlen essentielle Aminosäuren gerät die Eiweißsynthese ins Stocken und als Folge davon stellen sich lebensbedrohliche Mangelerscheinungen ein. In Peptiden und Proteinen unterschiedlicher Herkunft kommen die Aminosäuren nicht immer im gleichen Verhältnis vor. Teilweise sind darin nicht alle Aminosäuren zugleich vorhanden.

Unter allen Nahrungsmitteln kann bei der Ernährung auf Proteine am wenigsten verzichtet werden So lässt sich durch gesteigerte Eiweißverbrennung der Ausfall an Fett und Kohlenhydraten für einige Zeit ausgleichen, dagegen führt fehlende Proteinzufuhr selbst bei überreichlicher Zufuhr an Fett und Kohlenhydraten nach kurzer oder langer Zeit zu tödlicher Auszehrung, da der Erwachsene täglich ca. 30 Gramm seines Körpereiweißes verbrennt.

Verschiedene Proteine in der Nahrung sind wegen unterschiedlicher Aminosäurenzusammensetzung für den Menschen nicht biologisch gleichwertig. Der Mensch kann zum Beispiel aus Fleisch mehr körpereigene Substanz aufbauen als etwa aus Weizen- oder Maisproteinen. Diese Biologische Wertigkeit wird in Zahlenwerten angegeben, wobei die für Vollei willkürlich auf 100 festgesetzt wurde. Je höher die Biologische Wertigkeit, desto mehr körpereigene Substanz kann aus einem Protein aufgebaut werden.

Über den Verdauungstrakt mit der Nahrung zugeführte Proteine werden im Magen und Darm unter Einfluss eiweißspaltender Enzyme zu Peptiden und Aminosäuren abgebaut. Die Spaltprodukte wandern dann durch die Darmwand und werden in den Zellen nach erfolgter Desamierung entweder zu Kohlendioxid und Wasser oxidiert, oder aber mit Hilfe von Nucleinsäuren und Enzymen zu arteigenen Eiweißstoffen zusammengefügt.

Bei der Protolyse innerhalb der Zellen spielen auch Lysosomen und die darin enthaltenen Kathepsine eine wichtige Rolle, was dazu führt, dass Proteine, die bestimmte Aminosäuresequenzen enthalten, schneller abgebaut werden. Es hat sich herausgestellt, dass vor allem Oligopeptide vom Körper schnell aufgenommen werden können, während sehr große Proteine und auch freie Aminosäuren weniger schnell aufgenommen werden.

Die kommerziell erhältlichen Proteinprodukte haben den Nachteil, dass die Aminosäurenzusammensetzung häufig nicht der Zusammensetzung entspricht, die vom Körper benötigt wird. Auch die biologische Wertigkeit der bekannten Proteinprodukte ist meist geringer als erwünscht.

Proteine sind demzufolge nicht einheitlich in ihrer Wirkung. So hat es sich in den letzten Jahren herausgestellt, dass verzweigtkettige Aminosäuren (BCAA), also Leucin, Isoleucin und Valin, einen anderen Metabolismus im Körper als die übrigen Aminosäuren aufweisen.

So findet der Metabolismus von BCAA über die Skelettmuskulatur statt, während die anderen Aminosäuren in der Leber unmittelbar über die Gluconeogenese zu Glukose abgebaut werden. Demgegenüber wird die Muskelproteinsynthese üblicherweise über die Kette Leucin/Insulin gesteuert. Beim Abbau der BCAA findet zunächst eine Transaminierung und erst dann der Aufbau der desaminierten Carbonsäuren statt.

Molkenprodukte mit einem verbesserten Gehalt an zweikettigen Aminosäuren haben den Nachteil, dass die Balance zwischen den Aminosäuren noch nicht ausgeglichen erscheint, da die durch die Umsetzung der übrigen Aminosäuren auftretenden Metaboliten, wie Purine, nachteilig sind und zu Stoffwechselstörungen fuhren können.

Aus der EP 48 27 15 sind Zusammensetzungen für Nährzwecke bekannt, die neben proteinfreien Kohlenhydraten und speziellen proteinfreien polyungesättigten Fetten auch Aminosäurezusammensetzungen enthalten können. Diese Aminosäurezusammensetzungen bestehen aus einzelnen Aminosäuren, die für übliche Konsumenten aufgrund ihres üblen Geschmacks nicht essbar und darüber hinaus auch zu teuer sind, so dass diese allenfalls für hochspezielle Einzelanwendungen eingesetzt werden. Per os werden diese Zusammensetzungen nur per Sonde oder per Kapsel und dergleichen von Patienten eingenommen.

Auch die EP 15 82 207 beschreibt aminosäurehaftige Diäten, jedoch keine Diäten auf Proteinbasis. Insofern gilt auch hier das Vorhergesagte.

Ferner ist in der EP 14 79 300 die Herstellung eines Trockenpulvers von Aminosäuren mittels eines speziellen Verfahrens für bestimmte Partikelgrößen beschrieben, die wiederum als medizinische Diäten eingesetzt werden sollen. Nicht beschrieben sind Proteine mit einem speziellen Gehalt an BCAA.

Des Weiteren ist aus der US-A-5242697 eine mit essentiellen Aminosäuren angereicherte Zusammensetzung bekannt, die als Supplement zur Erhöhung des Nährwerts von Lebensmitteln verwendbar ist. Überdies offenbart die WO-A1-200449830 eine Zusammensetzung zur anhaltenden Erholung von Muskelermüdung, umfassend die Aminosäuren Leucin, Isoleucin, Valin und Glutamin und eine Molkenproteinkomponente, und Lebensmittel, welche diese Zusammensetzung als Wirksubstanz enthalten. Nicht beschrieben sind Proteinzusammensetzungen mit einem speziellen BCAA-Gehalt.

Der Erfindung liegt daher die Aufgabe zu Grunde, ein diätetisches Nahrungsmittel auf der Basis von Proteinen bereit zu stellen, die eine verbesserte Stickstoffbalance im Körper erzeugen.

Weiterhin liegt der Erfindung die Aufgabe zu Grunde, ein diätetisches Produkt auf der Basis von Proteinen zur Verfügung zu stellen, das kostengünstig herzustellen ist und darüber hinaus einen guten Geschmack aufweist.

Eine weitere Aufgabe der Erfindung ist es, eine Proteinzusammensetzung zur besseren Behandlung physiologischer Proteinmangelzustände zu liefern.

Weiter ist Aufgabe der Erfindung, eine Proteinzusammensetzung zur Behandlung von Proteinmangelzuständen zu liefern, die eine Aminosäurezusammensetzung aufweist, welche vom Körper benötigt wird und leicht vom Körper aufgenommen werden kann.

Weiter ist Aufgabe der Erfindung, eine Proteinzusammensetzung zur Behandlung von Proteinmangelzuständen zu liefern, die eine hohe biologische Wertigkeit aufweist.

Die Lösung dieser Aufgabe erfolgt durch die Maßnahmen der Ansprüche 1 bis 11.

Erfindungsgemäß wird eine Proteinzusammensetzung zur Behandlung physiologischer Zustände zur Verfügung gestellt, die leicht vom Körper aufgenommen werden kann.

Weiter wurde eine Proteinzusammensetzung zur Behandlung physiologischer Zustände gefunden, die eine hohe biologische Wertigkeit aufweist.

Unter "physiologisch" ist der gesunde Zustand eines Menschen zu verstehen, der einen klinisch unauffälligen Proteinmehrbedarf hat. Dieser Bedarf umfasst alle nicht krankhaft bedingten Proteinmehrbedarfszustände eines Menschen oder eines Saugetiers, beispielsweise Proteinmehrbedarfszustände durch Muskelaufbau bei Sport, insbesondere Kraftsport, Proteinmehrbedarfszustände bei intensivern Ausdauersport und Proteinmehrbedarfszustände bei kalorienreduzierten Diäten zur Gewichtsabnahme (bis zu einem body mass unterhalb 35) oder Diäten im Rahmen von Diabetes Typ II. Physiologisch bedingter, klinisch unauffälliger Proteinmehrbedarf ist dabei ein solcher, der nicht von Ärzten therapiert werden muss.

Die Aminosäuren in der erfindungsgemäßen Proteinzusammensetzung können als Oligopeptide, bestehend aus 2 bis 10 kondensierten Aminosäuren, als Polypeptide, bestehend aus 11 bis ca. 100 kondensierten Aminosäuren und als höhermolekulare Proteine, bestehend aus mehr als 100 kondensierten Aminosäuren, vorliegen. Bevorzugt sind Proteinzusammensetzungen, in denen mindestens 10 Gewichtsprozent aus Oligopeptiden und Polypeptiden, bezogen auf das Gesamtgewicht, der Oligopeptide, der Polypeptide und der höhermolekularen Proteine, bestehen. In einer besonders bevorzugten Ausführungsform beträgt dieser Anteil mindestens 20 Gewichtsprozent, noch bevorzugter mindestens 40 Prozent und insbesondere mindestens 60 Gewichtsprozent.

Eine erfindungsgemäße Proteinzusammensetzung besteht nach einer Analyse nach Kjeldal vorzugsweise aus mindestens 80 Gewichtsprozent, bezogen auf das Trockengewicht der Proteinzusammensetzung, besonders bevorzugt aus mindestens 85 Gewichtsprozent und insbesondere aus mindestens 90 Gewichtsprozent aus Oligopeptiden, Polypeptiden und hochmolekularen Proteinen.

Die verwendeten Aminosäuren schließen alle proteineigenen Aminosäuren ein. Bevorzugt weist eine erfindungsgemäße Proteinzusammensetzung zur Nahrungsergänzung alle essentiellen Aminosäuren auf. Besonders bevorzugt weist eine alle solche Proteinzusammensetzung zumindest alle essentiellen und weitere, nicht essentielle, proteineigenen Aminosäuren auf, und insbesondere weist eine solche Proteinzusammensetzung zumindest die Aminosäuren Ala, Arg, Asp, Cys, Glu, Gly, His, Ile, Leu, Lys, Met, Phe, Pro, Ser, Thr, Trp, Tyr und Val auf.

Die verzweigtkettigen Aminosäuren, die im Rahmen dieser Erfindung verwendet werden, sind Isoleucin, Leucin und Valin. Das Gewichtsverhältnis von Isoleucin : Leucin ist dabei von 1 : 1,4 bis 1 : 2,5 und das Verhältnis von Isoleucin : Valin von 1 : 1 bis 1 : 2.

Besonders bevorzugt ist ein Verhältnis von Isoleucin : Leucin von 1 : 1,4 bis 1 : 2 und das Verhältnis von Isoleucin : Valin von 1 : 1,1 bis 1 : 1,7. Insbesondere ein Verhätnis von Isoleucin zu Leucin von 1:1,35 bis 1: 1, 39 und von Isoleucin zu Valin von 1 :1,69 bis 1: 1,73 ist bevorzugt.

Weiter bevorzugt ist, dass eine erfindungsgemäße Proteinzusammensetzung eine aus Molke und/oder Milch gewonnene Proteinzusammensetzung ist. Besonders bevorzugt ist, dass die Proteinzusammensetzung unter Anwendung von Ultrafiltrationstechniken und/oder Mikrofiltrationstechniken aus Molke gewonnen worden ist. Als Molkenproteine liegen vor: $\beta$-Lactoglobulin (BCAA: 25 Gew.-%/100 g Protein), $\alpha$-Lactalbumin (21 %), Glycomacropeptide (22,5 %) sowie Serumalbumin, Immunoglobulin und Lactoferrin. In der Milch sind weiterhin enthalten die Kaseine mit einem BCAA-Gehalt von etwa 23 Gew.-%. Diese Proteine können einzeln oder in einem beliebigen Gemisch eingesetzt werden.

Proteingemische lassen sich nach üblichem Separationsverfahren trennen, beispielsweise durch Konzentrierung und Fraktionierung in RO-Filtrationsprozessen (Umkehrosmose) und durch selektive Fällverfahren.

Bei der RO-Membranfiltration werden die einzelnen Proteine in Abhängigkeit von ihren Molekulargewichten, also ihren Proteingrößen voneinander getrennt, wobei Membranen zum Einsatz kommen, die beispielsweise $\alpha$-Lactalbumin und Immunoglobulin in Folge ihrer großen Unterschiede der molekularen Massen trennen können. Andererseits können auch Proteine infolge ihrer kombinierten Unterschiede in Gewicht und Größe voneinander getrennt werden.

Hierbei ist es hilfreich, wenn die Ionenstärke und der pH-Wert der zu trennenden Molkenproteinlösung sorgfältig eingestellt werden. Insbesondere die pH-Werteinstellung ist von wesentlicher Bedeutung hinsichtlich des isoelektrischen Punkts der jeweiligen Proteine, bei dem das jeweilige Protein ausfällt.

Ähnliches gilt für die Einstellung der Elektrolytkonzentrationen, also der Ionenstärke, die die elektrostatische und sterische Abstoßung der Proteine an der Membran erhöhen können.

Durch diese vielfache Einstellungsmöglichkeit hinsichtlich pH-Wert und Ionenstärke können also Molkenproteine in ihre fraktionierten Proteinanteile zerlegt werden.

Ein Beispiel ist die chemische Ausfällung am isoelektrischen Punkt durch Einstellung des pH-Werts bei niedrigen Ionenstärken, bei der die betroffen Proteine ausfallen, während die anderen Proteine in Lösung bleiben, so dass beispielsweise $\alpha$-Lactalbumin durch Einstellung des pH-Werts auf 4,2 unter Denaturierung und Aggregation ausfällt, während die überstehende Lösung in den restlichen Proteinen, wie $\beta$-Lactoglobulin angereichert wird (vgl. Australian J. Dairy Technol. 38:144-149 (1983).

Andererseits kann aber auch bei einem pH-Wert von 4,65 durch Elektrodialyse $\beta$-Lactoglobulin ohne Erwärmen ausgefällt werden.

Man erhält also durch selektive Fällverfahren eine Fraktionierung ausgefällter Proteine einerseits und im Überstand enthaltener Proteine andererseits, die sich wiederum durch Änderung des isoelektrischen Punkts und/oder durch Veränderung der Ionenstärke bzw. Wärmebehandlung ebenfalls ausfällen lassen.

Insgesamt ist also durch fraktionierte Fällung ebenfalls eine Trennung der Molkenproteine in ihren Bestandteilen im Wesentlichen möglich.

Durch weiteren selektiven Abbau mittels Hydrolyse oder enzymatische Spaltung gelingt es, Molkenproteine im Speziellen so herzustellen, dass der BCAA-Gehalt auf mindestens 27,5 %, vorteilhafterweise mindestens 30 % und mehr gesteigert werden kann. Molkenproteine sind von mehreren Herstellern erhältlich, beispielsweise von der Firma Volactive, GB, Ross, USA oder Danmark Protein, Dänemark.

Aufgrund ihrer Spaltung mittels Hydrolyse oder durch Enzymreaktion in ihre Proteinfragmente haben diese Proteine den Vorteil, dass die Viskosität dieser Lösungen bei gleicher Proteinmenge erheblich sinkt, häufig um den Faktor 10, so dass sich wässrige Lösungen aufgrund der geringeren Viskosität erheblich besser durch Sonden an Patienten verabreichen lassen.

Proteine haben gegenüber kristallinen Aminosäuren den Vorteil, dass sie aufgrund ihres üblicherweise guten Geschmacks von allen Patienten bei oraler Einnahme auch in größeren Mengen akzeptiert werden. Demgegenüber sind Gemische aus einzelnen Aminosäuren in größeren Mengen praktisch nicht per os an Patienten aufgrund ihres üblen Geschmacks einzelner Aminosäuren zu verabreichen und werden von diesen auch bei Zugabe von Geschmacksverbesserern abgelehnt.

Es kommt hinzu, dass Gemische von einzelnen Aminosäuren infolge ihrer hohen Kosten für die diätetischen Zwecke nicht einsetzbar sind. Infolgedessen bleibt die Gabe von Aminosäuren in kristalliner Form weitgehend auf parenterale Lösungen für Schwerkranke beschränkt, die unmittelbar in die Blutbahn verabreicht werden.

Normale Individuen können jedoch nicht mit größeren Aminosäuremengen sowohl wegen der Kosten als auch wegen des üblen Geschmacks ernährt werden. Diätetische Zusammensetzungen auf Aminosäurebasis sind daher im Wesentlichen auch natürliche Proteine und deren Isolate sowie Abbauprodukte hiervon beschränkt, wobei nur Aminosäuren zugesetzt werden können, deren Geschmack akzeptabel ist. Zu letzteren gehören die verzweigtkettigen Aminosäuren Leucin, Isoleucin und Valin.

Demzufolge beschreibt der Stand der Technik nur BCAA-Diäten auf Aminosäurebasis, die seit den grundlegenden Erkenntnissen von Fischer Mitte der Siebzigerjahre bekannt sind und zu parenteralen Verabreichungen vorgesehen sind.

Erfindungsgemäß einsetzbare "Proteine" sind sämtliche Proteine, insbesondere Molkenproteine sowie Derivate hiervon, die mindestens einen Gehalt an BCAA von 27,5 g/100 g Protein aufweisen. Bevorzugt sind Proteine, die mindestens einen BCAA-Gehalt von 30 Gew.-% aufweisen. Der obere Gehalt von BCAA in den Proteinen übersteigt 40 Gew.-% nicht und sollte vorteilhafterweise unter 35 Gew.-% bleiben. Sollten diese Proteine als Ausgangsmaterial nicht den vorstehend genannten BCAA-Gehalt aufweisen, werden derartigen Proteinen so viel kristalline BCAA, also Isoeucin, Leucin und Valin, zugegeben, bis die angegebene Untergrenze von 27,5, vorzugsweise 30 Gew.-% erreicht ist. Die Zugabe kristalliner BCAA ist jedoch auf maximal 6 Gew.-% beschränkt. Vorteilhafterweise erfolg die Zugabe in einer solchen Weise, dass deren relatives Muster mit dem BCAA-Muster des Ausgangsproteins übereinstimmt, d.h. die relativen Verhältnisse der BCAA ändern sich dadurch nicht. Per Definition fallen solche Protein/AS-Gemische unter die vorstehenden "Proteine".

Weiter bevorzugt ist, dass die erfindungsgemäße Proteinzusammensetzung eine hervorragende Löslichkeit in Wasser besitzt. Besonders bevorzugt ist eine Löslichkeit bei 20 °C von mindestens 5 Gewichtsprozent Protein, insbesondere von mindestens 8 Gewichtsprozent.

Löslichkeit ist dabei so definiert, dass bei einer Schichtdicke von 1 cm die Transmission von weißem Licht einer Lösung der Protemzusammensetzung bei 20 °C maximal 10 Prozent geringer ist als die von destilliertem Wasser.

Die biologische Wertigkeit der Nahrungseiweiße gibt an, wie wertvoll das Eiweiß eines bestimmten Nahrungsmittel bzw. einer Proteinzusammensetzung für den Aufbau von Muskulatur und anderen körpereigenen Eiweißstoffen (z.B. Enzyme, Hormone) ist. Als Bezugswert dient das Vollei-Protein, denn ihm ist eine Biologische Wertigkeit von 100 gegeben worden. Alle anderen Proteine werden zu Vollei-Protein in Relation gesetzt.

Die Biologische Wertigkeit im Rahmen der Erfindung wird mit Hilfe des Essentiell Amino Acid Index (EAA-Index) bestimmt. Dieser errechnet sich folgendermaßen:

$$(EAA - Index) = \sqrt[n]{\left(\frac{Ile_T}{Ile_R} \bullet 100\right) \bullet \left(\frac{Leu_T}{Leu_R} \bullet 100\right) \bullet \left(\frac{Lys_T}{Lys_R} \bullet 100\right) \ldots \bullet \left(\frac{Val_T}{Val_R} \bullet 100\right)}$$

T=Testprotein, R=Referenzprotein, n=Anzahl der berücksichtigten Aminosäuren

Diese Formel bedeutet, dass man den Anteil einer essentiellen Aminosäure im zu beurteilenden Protein (Testprotein) ins Verhältnis setzt zum Anteil dieser Aminosäure in einem so genannten Referenzprotein (ein Protein hoher Biologischer Wertigkeit, die genau bekannt ist, z.B. Vollei). Multipliziert man diese n Werte miteinander und zieht die n-te Wurzel daraus, erhält man den Essentiell Amino Acid-Index (EAA-Index). Aus dem EAA-Index kann man nach Oser die Biologische Wertigkeit (BW) wie folgt berechnen:

$$BW = (EAA\text{-}Index \times 1,09) - 11,7$$

Es ist bevorzugt, wenn eine erfindungsgemäße Proteinzusammensetzung eine biologische Wertigkeit von größer 135 aufweist, besonders bevorzugt größer 140, insbesondere größer 145.

Die Proteine bzw. Protein/BCAA-Gemische können, sofern es zweckmäßig erscheint, auch mit Kohlenhydraten, Vitaminen, anorganischen Salzen und Spurenelementen, versehen sein.

Als Vitamine können Vitamin C, Niacin, Vitamin $B_1$ und $B_2$, Folsäure, Biotin, Pantothensäure, Cholin, Vitamin A, Vitamin D oder Vitamin E zugesetzt sein. Desgleichen können übliche Elektrolyte, wie Calcium, Magnesium, Natrium und Kaliumsalze zugesetzt werden Zum Einsatz kommen also solche Vitamine und Elektrolyte, wie sie üblicherweise bei diätetischen Nahrungsmitteln verwendet werden.

Die Gemische werden nach den üblichen Techniken hergestellt, beispielsweise durch Vermischen wässriger Lösungen der einzelnen Komponenten, Sprühtrocknen oder Vakuumtrocknen und anschließendes Verkleinern des getrockneten Produkts auf eine vorbestimmte Korngröße.

Derartige Pulver können direkt als pulverförmige Nahrungsmittel eingesetzt werden oder aber kommen als Flüssignahrungsmittel mit entsprechender Rezeptur in den Handel.

Die erfindungsgemäße Proteinzusammensetzung weist eine gute Löslichkeit und eine geringe Viskosität auf.

Bevorzugt wird die erfindungsgemäße Proteinzusammensetzung dem Patienten in Form von Proteinriegeln und/oder löslicher Getränkepulver verabreicht.

Beispiele:

Die folgenden Beispiele offenbaren beispielhafte Aminosäurezusammensetzungen von erfindungsgemäßen Proteinzusammensetzungen, die aus Molke unter sauren Bedingungen unter Verwendung von Ultrafiltrationstechniken und Mikrofilirationstechoiken gewonnen wurden. Diese sollen den Gegenstand der Erfindung allerdings nicht einschränken.

Beispiele 1-3

In den Beispielen 1 bis 3 beträgt der Gehalt an Beta- Lactoglobulin von 39 bis 48 Gewichtsprozent, der Gehalt an Alpha Lactalbumin von 12 bis 18 Gewichtsprozent, der Gehalt an Bovinem Serumalbumin von 1 bis 2 Gewichtsprozent, der Gehalt an Imunglobulin G von 1 bis 3 Gewichtsprozent, der Gehalt an Lactoferrin weniger als ein Gewichtsprozent und der Gehalt an Glycomakropeptid von 22 bis 28 Gewichtsprozent, bezogen auf 100 Gewichtsprozent Proteine

Die Aminosäurenzusammensetzungen der Proteinzusammensetzungen sind in Tabelle 1 angegeben.

Tabelle 1

| Aminosäureanteile der Proteinzusammensetzungen aus Beispiel 1, 2 und 3 in Gewichtsprozent, bezogen auf das Gesamtgewicht der Oligopeptide, Polypeptid und hochmolekularen Proteine. | | | |
|---|---|---|---|
| | Beispiel 1 | Beispiel 2 | Beispiel 3 |
| Ala | 4,5 | 4,3 | 4,2 |
| Arg | 1,9 | 1,8 | 1.7 |
| Asp | 10,0 | 9,6 | 9,3 |
| Cys | 2,0 | 1,9 | 1,8 |
| Glu | 16,4 | 15,7 | 15,2 |
| Gly | 1,3 | 1,2 | 1,2 |
| His | 1,5 | 1,4 | 1,4 |
| Ile | 7,4 | 8,2 | 8,7 |
| Leu | 12,7 | 14,0 | 14,8 |
| Lys | 8,7 | 8,3 | 8,1 |
| Met | 2,0 | 1,9 | 1,8 |
| Phe | 2,7 | 2,6 | 2.5 |
| Pro | 5,0 | 4,8 | 4,7 |
| Ser | 4,2 | 4,0 | 3,9 |
| Thr | 6,0 | 5,7 | 5,5 |
| Trp | 1,3 | 1,2 | 1,2 |
| Tyr | 2,3 | 2,2 | 2,1 |
| Val | 10,2 | 11,2 | 11,9 |
| Summe | 100,0 | 100,0 | 100,0 |

(fortgesetzt)

| Aminosäureanteile der Proteinzusammensetzungen aus Beispiel 1, 2 und 3 in Gewichtsprozent, bezogen auf das Gesamtgewicht der Oligopeptide, Polypeptid und hochmolekularen Proteine. | | | |
|---|---|---|---|
| | Beispiel 1 | Beispiel 2 | Beispiel 3 |
| | | | |
| Summe BCAA | 30,4 | 33,4 | 35,4 |
| | | | |
| Anteil Ile | 0,24 | 10,24 | 0,24 |
| Anteil Leu | 0.42 | 0,42 | 0,4 |
| Anteil Val | 0,34 | 0,34 | 0,34 |

Beispiel 4

Ein Molkenproteinkonzentrat (Danmark Protein A/S, Dänemark) mit einem BCAA-Gehalt von 24,4 Gew.-% wird als Ausgangsmaterial eingesetzt, wobei das Protein 6,5 Gew.-% Valin, 6,7 Gew.-% Isoleucin und 11,2 Gew.-% Leucin aufweist. Diesem Protein werden 1,8 g Leucin, 1,2 g Isoleucin und 1,1 g Valin zugesetzt, so dass der Endgehalt des Protein/AS-Gemisches einen BCAA-gehalt von 28,5 Gew.-% aufweist.

Beispiel 5

Das Ausgangsprotein von Beispiel 4 wird mit 3 g Leucin sowie jeweils 1,5 g Isoleucin und Valin, jeweils bezogen auf 100 g, angereichert. Demzufolge besitzt dieses Proteingemisch einen BCAA-Gehalt von 30,4 g/100 g Protein. Es hat eine Löslichkeit in Wasser von 8 Gew.%.

Beispiel 6

Die Protein- bzw. Protein/Aminosäuregemische gemäß den Beispielen 1 bis 5 werden zu Diätetika verarbeitet, die je 100 g einen Brennwert von 14 kJ, entsprechend 3,33 kcal, aufweisen.

Diese Proteine weisen als Elektrolytzusatz folgende Salze auf: 2 g Ca, 0,4 g Mg, 1,1 g P, 14 mg Fe. An Vitaminen enthält das Konzentrat 0,2 g Vitamin C, 25 mg Niacin, 25 mg Vitamin E, 10 mg Pantothensäure, 2,5 mg Vitamin $B_6$, 2 mg Vitamin $B_1$, 2,3 mg Vitamin $B_2$, 0,8 mg Folsäure sowie 9 μg Vitamin $B_{12}$.

Diese Elektrolyt- und Vitaminbeigaben sind jeweils in 80 g Protein enthalten, das in Pulverform vorliegt. Weiterhin enthält die Zusammensetzung Geschmacksbildner in Form von Vanille-, Schoko- oder Himbeergeschmack.

Eine Mahlzeit enthält 25 g der Zusammensetzung und wird entweder in 250 ml Wasser oder 250 ml 0,3 %iger Milch vermischt und anschließend getrunken.

**Patentansprüche**

1.   Diätetische Zusammensetzung zur Behandlung von physiologischen Proteinmangelzuständen, umfassend eine Proteinzusammensetzung, enthaltend Proteine und bis zu 6 Gew.-% der kristallinen verzweigtkettigen Aminosäuren Isoleucin, Leucin und Valin, wobei der Gesamtgehalt der in der Proteinzusammensetzung enthaltenen verzweigtkettigen Aminosäuren Isoleucin, Leucin und Valin mindestens 27,5 Gew.-% bis maximal 40 Gew.-% beträgt, und wobei das Gewichtsverhältnis der verzweigtkettigen Aminosäuren Isoleucin:Leucin 1:1,4 bis 1:2,5 und das Gewichtsverhältnis der verzweigtkettigen Aminosäuren Isoleucin:Valin 1:1 bis 1:2 beträgt.

2.   Diätetische Zusammensetzung nach Anspruch 1, wobei die Proteine Molkenproteine und/oder Milchproteine sind.

3.   Diätetische Zusammensetzung nach Anspruch 2, wobei die Molkenproteine β-Lactoglobulin, α-Lactalbumin und/ oder Glucomacropeptide und die Milchproteine Kaseine, einzeln oder im Gemisch, sind.

4.   Diätetische Zusammensetzung nach einem der Ansprüche 1 bis 3, wobei das Gewichtsverhältnis der verzweigtkettigen Aminosäuren Isoleucin:Leucin 1:1,4 bis 1:2 und das Gewichtsverhältnis der verzweigtkettigen Aminosäuren Isoleucin:Valin 1:1,1 bis 1:1,7 beträgt.

**5.** Diätetische Zusammensetzung nach einem der Ansprüche 1 bis 4, die durch eine biologische Wertigkeit von mindestens 140 **gekennzeichnet** ist.

**6.** Diätetische Zusammensetzung nach einem der Ansprüche 1 bis 5, die durch eine Löslichkeit in Wasser bei Raumtemperatur von mindestens 5 Gew.-%, vorzugsweise mindestens 8 Gew.-%, **gekennzeichnet** ist.

**7.** Diätetische Zusammensetzung nach einem der Ansprüche 1 bis 6, die ferner übliche Elektrolyte, Vitamine, Spurenelemente und/oder Geschmacksbildner umfasst.

**8.** Diätetische Zusammensetzung nach einem der Ansprüche 1 bis 7, im Rahmen von Proteinmehrbedarfszuständen bei Muskelaufbau bei Sport.

**9.** Diätetische Zusammensetzung nach einem der Ansprüche 1 bis 7, im Rahmen von Proteinmehrbedarfszuständen bei intensivem Ausdauersport.

**10.** Diätetische Zusammensetzung nach einem der Ansprüche 1 bis 7, im Rahmen von Proteinmehrbedarfszuständen bei kalorienreduzierten Diäten zur Gewichtsabnahme.

**11.** Diätetische Zusammensetzung nach einem der Ansprüche 1 bis 7, im Rahmen von Proteinmehrbedarfszuständen bei Diäten im Rahmen von Diabetes Typ II.

**Claims**

**1.** Dietetic composition for the treatment of physiological protein deficiency conditions, comprising a protein composition containing proteins and up to 6 wt.% of the crystalline branched-chain amino acids isoleucine, leucine and valine, wherein the total content of the branched-chain amino acids isoleucine, leucine and valine present in the protein composition is at least 27.5 wt.% to at most 40 wt.%, and wherein the weight ratio of the branched-chain amino acids isoleucine:leucine is 1:1.4 to 1:2.5 and the weight ratio of the branched-chain amino acids isoleucine: valine is 1:1 to 1:2.

**2.** Dietetic composition according to Claim 1 wherein the proteins are whey proteins and/or milk proteins.

**3.** Dietetic composition according to Claim 2 wherein the whey proteins are β-lactoglobulin, α-lactalbumin and/or glucomacropeptides, and the milk proteins are caseins, individually or in a mixture.

**4.** Dietetic composition according to one of Claims 1 to 3 wherein the weight ratio of the branched-chain amino acids isoleucine:leucine is 1:1.4 to 1:2 and the weight ratio of the branched-chain amino acids isoleucine:valine is 1:1.1 to 1:1.7.

**5.** Dietetic composition according to one of Claims to 4 which is **characterized by** a biological value of at least 140.

**6.** Dietetic composition according to one of Claims 1 to 5 which is **characterized by** a solubility in water at room temperature of at least 5 wt.%, preferably of at least 8 wt.%.

**7.** Dietetic composition according to one of Claims 1 to 6 which further comprises conventional electrolytes, vitamins, trace elements and/or flavourings.

**8.** Dietetic composition according to one of Claims 1 to 7 in the context of conditions of increased protein demand for muscle building in sport.

**9.** Dietetic composition according to one of Claims 1 to 7 in the context of conditions of increased protein demand in intensive stamina sports.

**10.** Dietetic composition according to one of Claims 1 to 7 in the context of conditions of increased protein demand associated with reduced-calorie diets for weight loss.

**11.** Dietetic composition according to one of Claims 1 to 7 in the context of conditions of increased protein demand associated with diets for type 2 diabetes.

## Revendications

**1.** Composition diététique pour le traitement d'états de carences protéiques physiologiques, comprenant une composition de protéine, contenant des protéines et jusqu'à 6 % en poids des acides aminés à chaîne ramifiée cristallins isoleucine, leucine et valine, la teneur totale des acides aminés à chaîne ramifiée isoleucine, leucine et valine contenus dans la composition de protéine étant d'au moins 27,5 % en poids à 40 % en poids au maximum, et dans laquelle le rapport en poids des acides aminés à chaîne ramifiée isoleucine : leucine étant de 1 : 1,4 à 1 : 2,5 et le rapport en poids des acides aminés à chaîne ramifiée isoleucine : valine étant de 1 : 1 à 1 : 2.

**2.** Composition diététique selon la revendication 1, dans laquelle les protéines sont des protéines lactosérique et/ou des protéines lactiques.

**3.** Composition diététique selon la revendication 2, dans laquelle les protéines lactosériques sont la β-lactoglobuline, l'α-lactalbumine et/ou les glucomacropeptides et les protéines lactiques caséines, seules ou en mélange.

**4.** Composition diététique selon l'une des revendications 1 à 3, dans laquelle le rapport en poids des acides aminés à chaîne ramifiée isoleucine : leucine est de 1 : 1,4 à 1 ; 1,2 et le rapport en poids des acides aminés à chaîne ramifiée isoleucine : valine est de 1 : 1,1 à à 1 : 1,7.

**5.** Composition diététique selon l'une des revendications 1 à 4, qui est **caractérisée par** une valeur biologique d'au moins 140.

**6.** Composition diététique selon l'une des revendications 1 à 5, qui est **caractérisée par** une solubilité dans l'eau à température ambiante d'au moins 5 % en poids, de préférence, d'au moins 8 % en poids.

**7.** Composition diététique selon l'une des revendications 1 à 6, qui comprend en outre des électrolytes, vitamines, oligo-éléments et/ou agents de sapidité habituels.

**8.** Composition diététique selon l'une des revendications 1 à 7, dans le cadre des états de besoins accrus en protéines dans la croissance musculaire dans le cadre du sport.

**9.** Composition diététique selon l'une des revendications 1 à 7, dans le cadre des états de besoins accrus en protéines dans le sport d'endurance intensif.

**10.** Composition diététique selon l'une des revendications 1 à 7, dans le cadre des états de besoins accrus en protéines dans les régimes à teneur réduite en calories pour la perte de poids.

**11.** Composition diététique selon l'une des revendications 1 à 7, dans le cadre des états de besoins accrus en protéines dans les régimes alimentaires dans le cadre du diabète de type II.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 482715 A
- EP 1582207 A
- EP 1479300 A
- US 5242697 A
- WO 200449830 A1

**In der Beschreibung aufgeführte Nicht-Patentliteratur**

- *Australian J. Dairy Technol.,* 1983, vol. 38, 144-149